# EUROPEAN PATENT SPECIFICATION

(11) **EP 0 415 671 B1**
(45) Date of publication and mention of the grant of the patent: **29.03.1995**
(21) Application number: 90309318.5
(22) Date of filing: 24.08.1990
(51) Int. Cl.: A61K 9/22

(54) **Gastric retention device**
Gastrisches Zurückhaltungssystem
Système de rétention gastrique

(30) Priority: 31.08.1989 JP 225245/89
(43) Date of publication of application: 06.03.1991
(73) Proprietor: YAMANOUCHI PHARMACEUTICAL CO., LTD., Tokyo 103 (JP)
(72) Inventor: Sonobe, Takashi, Fujieda-shi, Shizuoka (JP); Watanabe, Shunsuke, Fujieda-shi, Shizuoka (JP); Katsuma, Masataka, Fujieda-shi, Shizuoka (JP); Takamatsu, Narushi, Yaizu-shi, Shizuoka (JP); Konno, Yutaka, Yaizu-shi, Shizuoka (JP); Takagi, Hirokazu, Yaizu-shi, Shizuoka (JP)
(74) Representative: Geering, Keith Edwin

(56) References cited:
- EP-A- 0 090 560
- EP-A- 0 202 159

## Description

The present invention relates to a device for extended retention in the stomach and more particularly but not exclusively to a device which can retain a drug in the stomach for a predetermined definite period of time and release the drug over or after passage of a definite time.

Known devices for retaining a drug in the stomach for a definite period after administration include the floating type comprising a composition (U.S.Patent No.4,126,672) of a gel-forming high mol.wt. substance (hydroxypropylmethyl cellulose, etc.) and hydrogenated cotton seed oil having carried thereon a drug and which is designed to have a specific gravity of 1 or less and so float in the stomach to increase the residence time of the drug in the stomach; the mucous membrane adhesion type - formulated with a component which is polyallylamine (for example, PAA-HC1-H manufactured by Nitto Spinning Co.,Ltd.) or an anionic high mol.wt. substance (for example, carboxyvinyl polymer) and adheres to mucous membrane (for example, Japanese Patent Application Laid-Open Nos.62-132830 and 62-77335), or comprising a composition of a water-soluble high mol.wt. substance (for example, polyethylene oxide, polyvinyl alcohol) and an oil (for example, medium chain triglyceride) whereby a gel layer is formed after administration to control release of the drug and retard mobility of the drug into the gastrointestinal tract by its adherence to mucous membrane; the swelling type obtained by compressing erodible polymer to a small size and enclosing it in a capsule, the polymer reverting to its original form (due to its elasticity) on dissolution of the capsule in the stomach after administration (e.g. Japanese Laid-Open Patent Application No.62-26215); and the fibre type using compressed or constrained fibres or ribbon (Japanese Patent Application Laid-Open Nos. 63-23815 and 2-29268).

With the floating type of device, even though its specific gravity is controlled, gastric residence time is unaffected but is influenced more by whether the subject if fasted or fed. [P.Gruber, et.al., Journal of Pharmaceutical Science, 76, 117-122 (1987); S.Sangekar, et.al., Indian Journal of Pharmacy, 35, 187,191 (1987)]. In the mucous membrane adhesion type, gastric residence time is short and residence time varies greatly. The swelling type and fibre type have the problems that both require a special fastener or constraint means to maintain the compressed state, that there is the danger of the constraint force being released prior to or during administration, and that plastic deformation might occur or elasticity might be diminished so that restoration to the original shape is not adequate.

The present invention provides a device for extended retention in the stomach comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach.

The present invention can thus provide a device which can ensure retention of a drug in the stomach for a definite period. The invention includes a device which can maintain a small-sized dosage form safely prior to administration and yet readily revert to the original form in the stomach after administration, by using a shape memory substance. The invention can provide easy control of gastric residence time, by selection of the material erodible in vivo and/or by a coating treatment.

The shape memory material may be a substance having crosslinked structure and crystal structure below its melting point, but which above its melting poiint is amorphous and plastic, or a substance having a glass transition point (Tg) at about room temperature and which has a large change in modulus of elasticity around Tg. Examples of such substances include polynorbornenes (containing an agent to prevent senile memory decay) and derivatives thereof (e.g. NOSOREX^{R}, manufactured by Nippon Zeon Co.,Ltd.); polyurethanes (e.g. DIARY^{R}, manufactured by Mitsubishi Heavy Industry Co., Ltd.); trans-polyisoprenes (KURAPRENE^{R}, manufactured by Kuraray Co.,Ltd); styrene type hybrid polymers (e.g. ASMER^{R}, manufactured by Asahi Chemical Industry Co.,Ltd.); ethylene-vinyl acetate copolymers; polycaprolactone; crosslinked polyethylene, polyamide; polyfluorinated vinylidene; etc. Of these, substances having a melting point or Tg close to body temperature with the maximum load around body temperature being 150 to 3000 g (measured by the method described in Reference Example 1) are preferred for the present invention.

In polyurethanes, Tg and physical properties can be varied by altering the isocyanate or polyol constituents, the chain extension agent, the formulation ratios, etc. The polyurethane used in the present invention may be urethane elastomer described in Japanese Laid-Open Patent Application No.1-264829 and mixtures thereof which have the physical properties described above.

The material erodible in vivo is a material which has a definite flexibility and strength but which upon contact with body fluids gradually disintegrates or swells or melts or dissolves to loose its shape. Examples of erodible substance which can be used in the present invention include polyethylene oxide, polyvinyl alcohol, soluble cellulose (HPC, HP-MC, MC (methyl cellulose), HPMCP, CAP, etc.), ethylene vinyl alcohol, ethylene-maleic acid copolymers, polyacrylate, methacrylic acid copolymer, a blend of methacrylic acid copolymer and aminoalkyl methacrylate copolymer, polycapronolactone, poly(ortho) ester, polyurethane, PVP, polylactone, polyamide, polypeptide, gelatin, polyacrylonitrile, polyester, etc. These substances may be used singly or in a suitable combination of two or more.

The device of the present invention is a molding comprising shape memory material and material erodible in vivo which are conjugated or mixed with each other. For purposes of improving adherence and controlling strength, suitable adhesives may also be used. The device adopts a shape and size in the stomach such as not to pass through the pylorus, but after the erodible substance is released the remains of the device can pass through the pylorus. The device of the present invention can be constructed with at least 3 coplanar limbs extending from a centre (e.g. it can be Y-shaped, cross-shaped, tetrapod type, etc.), or it can be ring-shaped or of circular or cylindrical shape. Preferably, the device provides a gap or gaps through which gastric contents can pass.

The invention is illustrated, by way of example, in the following description of embodiments to be read in conjunction with the accompanying drawings, in which :
FIGURES 1 and 2 show respectively Y-shaped and cruciform devices of the present invention; in these and subsequent FIGURES, (A), (B) and (C) denote respectively shape memory, in vivo erodible, and joint parts.
FIGURE 3 shows at (a), (b) and (c) three other devices according to the present invention, those at (a) and (b) being shown in planar and folded form;
FIGURE 4 shows a device according to the invention wherein the part erodible in vivo is coated. FIGURE 4(a) shows the folded state and FIGURE 4(b) shows the folded device encased in a capsule;
FIGURE 5 shows a manufacturing process for a Y-shaped device according to the invention, using an injection molding machine. Erodible parts (B) (Fig.5a) are placed in a metal mold (Fig.5b) and shape memory portion (A) is injected at the centre to join parts A and B to form the device;
FIGURE 6 shows a flow chart for preparing a Y-shaped device comprising a conjugate of shape memory and in vivo erodible materials, the joint part for which is formed by using a fusing agent; FIGURE 6(b) shows the shape memory portion (A) of Fig.6a and the parts (B) of erodible substance in a mold for injection molding; FIGURE 6(c) shows a Y-shaped device obtained after joining them by injecting fusing agent between the parts (A) and (B);

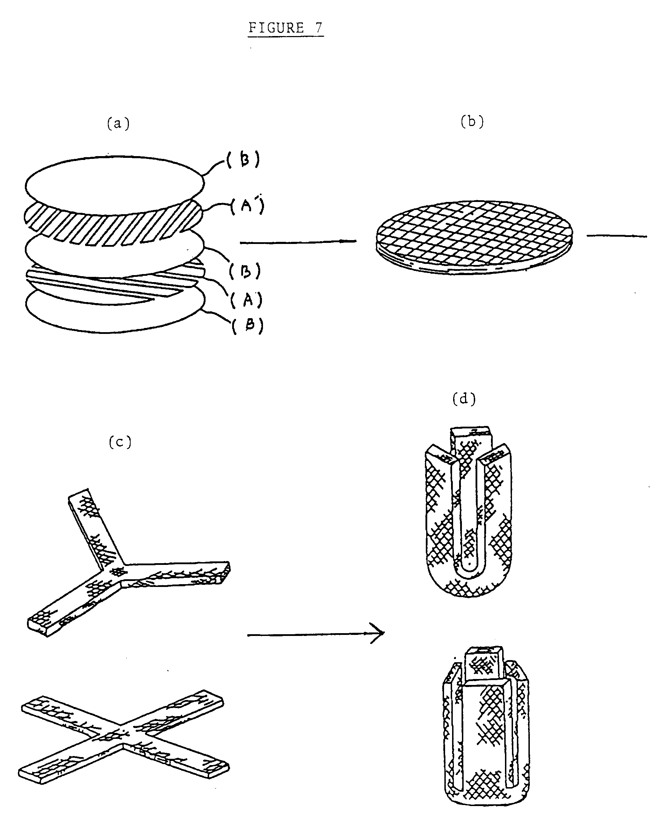

FIGURE 7 shows a flow chart for preparing cruciform and Y-shaped devices comprising a laminate of shape memory and erodible materials. In Fig.7, (A) represents a layer of shape memory material and (B) a thin layer of material erodible in vivo; FIGURE 7(a) shows two such layers (A) disposed lattice-like between three layers (B); FIGURE 7(b) shows a flat disk compression molded from the 7(a) assembly; FIGURE 7(c) shows cruciform and Y-shaped devices according to the invention cut from 7(b) disks; FIGURE 7(d) shows the latter devices folded for oral administration; one or both layer(s) (A) could instead be of one or more yarns of shape memory material, with no (or a restricted amount of) overlapping in the layer so that the latter on its own can exit from the stomach.
FIGURE 8 shows construction by compression molding of a Y-shaped device, comprising a conjugate of shape memory material (A) , erodible material (B) and fusing agent (C);
FIGURE 9 shows the plasma level of nicardipine hydrochloride as a function of time after orally administering to Beagle dogs the sustained release tablets and the devices prepared by Example 21. (The plasma level is a mean value ± SE in the case of 6 dogs);
FIGURE 10 shows the change with time of plasma level of nicardipine hydrochloride after orally administering to Beagle dogs ordinary tablets and the devices prepared by Example 21. For comparison, the results for the gastric retention device (containing 280 mg of the drug), which results are shown in Fig.9, are also shown in the Figure. (The plasma level is a mean value ± SE in the case of 6 dogs);
FIGURE 11 shows a device according to the invention to the erodible limb parts of which a drug-containing part (D) is attached;
FIGURE 12 shows the change with time of plasma level of nicardipine hydrochloride after orally administering to Beagle dogs the device prepared by Example 22 and conventional tablets prepared by Example 21 (a). (The plasma level is a mean value ± SE in the case of 6 dogs);
FIGURE 13 shows the change with time of plasma level of nicardipine hydrochloride after orally administering to Beagle dogs the device prepared by Example 23; and

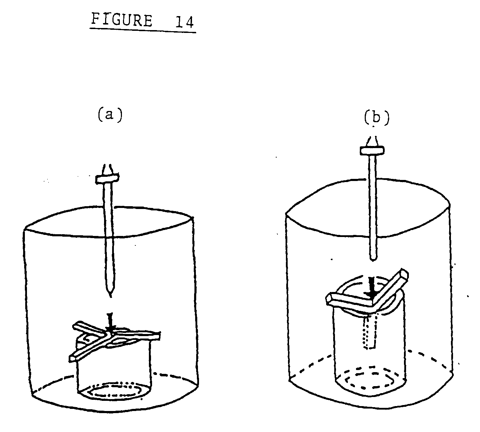

FIGURE 14 shows the test method in Reference Example 2 in the case of applying the maximum load in vertical and horizontal directions to the Y-shaped device.

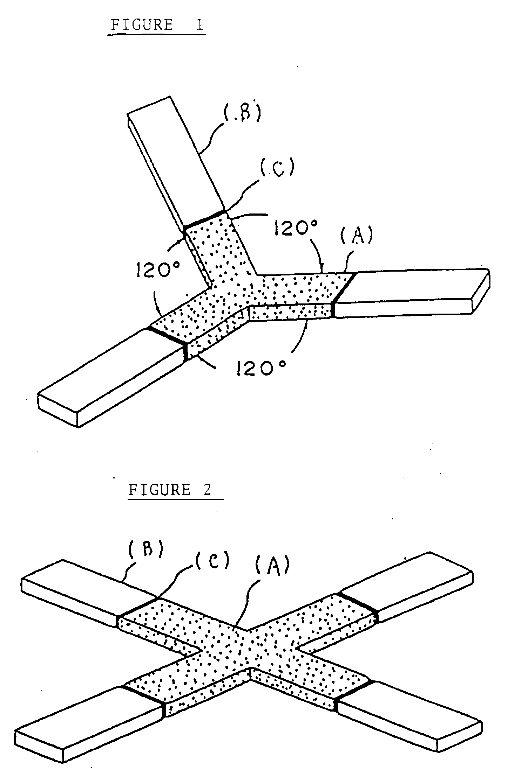

The devices shown in Figs.1 and 2 are Y-shaped or cross-shaped comprising a conjugate of shape memory and erodible materials.

Part (A) is of the shape memory material and since this part should be discharged from the stomach, its size can be as large as will still readily pass through the pylorus or other gastrointestinal tracts and is generally 2 cm or less, preferably about 0.5 to about 1.5 cm, in shorter diameter. The tip portion (B) of each limb is of material erodible in vivo. The length of this portion is e.g. 1 to 3 cm, which may give 2 to 7.1 cm as shorter diameter of the device as a whole. The joint part (C) is formed by physical meshing or with fusing agent or adhesive. The fusing agent is a substance which has a strong affinity to (A) and (B) and is appropriately chosen depending upon the latter materials. Examples of the fusing agent are polyurethane, ethylene vinyl acetate polymer, etc. As the adhesive, there may be used an epoxy resin or cyanoacrylate resin adhesive.

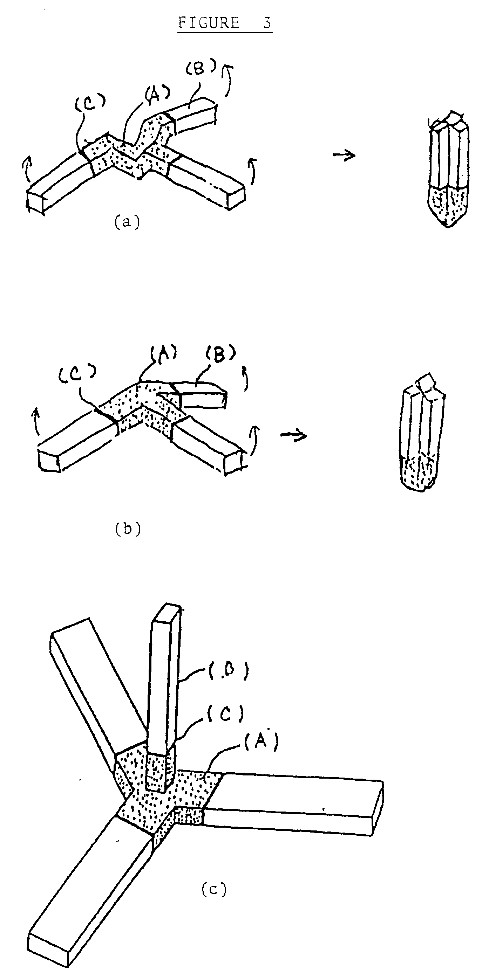

The device of the present invention must have such a strength or durability that it is retained in the stomach for a definite period of time, against gastric motility. For a Y-shaped or cross-shaped device having such a strength, a component showing a maximum load of 150 to 3000 g (when measured by the method described in Reference Example 1) at about body temperature may be used. In the case of using, for example, polynorbornene, it is advisable that the thickness be at least e.g. 0.5 mm, preferably about 2.8 mm, and the limb width at least 2 mm, preferably about 2.8 mm. This applies also to shapes such as shown in Fig.3(a) and Fig.3(b). In the arrow-shaped embodiment of Fig.3(b) , the outer limbs are for example at an angle of 60° to the central limb.

The device shown in Fig.3(c) has 4 extending limbs. At the central portion of the device, a shape memory material or a conventional pharmaceutically acceptable pillar-shaped substance is jointed - where shape memory material (A) is used, erodible substance (B) is further adhered to the tip portion.

The device shown in Fig.7 is an example of a molding comprising a laminate of the shape memory and erodible materials. This Y-shaped or cross-shaped device has the erodible material in three layers, between which strip- or yarn-like shape memory material is disposed lattice-like. The thickness of each erodible layer is suitably about 0.5 mm. It is appropriate that each shape memory layer has a thickness of about 0.5 mm and a width of about 2 mm. This shape memory material is disposed lattice-like at intervals of 0.5 mm to 2 mm between the thin layers made of the erodible material. The thus disposed shape memory and in vivo erodible layers are subjected to thermal compression molding followed by cutting to the required shape. The shaped laminate is disintegrated into small pieces of the shape memory material as gastric erosion proceeds and therefore eventual discharge from the digestive tract is easy.

Another construction of the device in accordance with the present invention is a molding comprising a homogeneous mixture of the shape memory and erodible materials. The mixing ratio of these substances is such that the restoring force of the shape memory material is maintained; for example, 1 to 4 parts by weight of the erodible material are used per 1 part of the shape memory material.

For purposes of controlling the erosion rate of the device it may be coated. The coating may be applied to the entire surface of the device or only to part(s) thereof, e.g. to an erodible part or parts. Any coating agent is usable so long as it is suitable for pharmaceutical preparations. Preferred examples of the coating agent include **EUDRAGIT RS** (trademark, manufactured by Rohm & Haas Co.,Ltd., component: 1 : 2 : 0.1 copolymer of ethyl acrylate, methyl methacrylate and trimethylammonioethyl methacrylate), **EUDRAGIT RS** (trademark, manufactured by Rohm & Haas Co.,Ltd., component: 1 : 2 : 0.1 copolymer of ethyl acrylate, methyl methacrylate and trimethylammonioethyl methacrylate), **EUDRAGIT L** (trademark, manufactured by Rohm & Haas Co.,Ltd., component: 1 : 1 copolymer of ethyl methacrylate and methyl methacrylate, **EUDRAGIT S** (trademark, manufactured by Rohm & Haas Co.,Ltd., component: 2 : 1 copolymer of methyl methacrylate and methacrylic acid),**EUDRAGIT L30D-55** (trademark, manufactured by Rohm & Haas Co.,Ltd., component: 1 : 1 copolymer of ethyl acrylate and methacrylic acid), **ETHOCEL** (trademark, manufactured by Dow Chemicals); **TC-5** (trademark, manufactured by Shin-Etsu Chemical Co., Ltd.. component: hydroxypropylmethyl cellulose), hydroxypropylmethyl cellulose phthalate (JP XI), cellulose acetate phthalate (JP XI), Shellac (JP XI), etc. These substances may be used singly or in appropriate mixtures thereof. If necessary and desired, a plasticizer may also be added thereto. Examples of the plasticizer include triacetin, MACROGOL 400, triethyl citrate, Tween 80, castor oil, etc.

The device of the present invention is administered by folding into a shape suited for administration. The device is folded at a site of the shape memory substance. In oral administration, the folded device may be administered as it is; alternatively, the device is enclosed in a capsule and the capsule is provided for administration. The folded device may also be coated with conventional coating agents such as hydroxypropylmethyl cellulose, hydroxypropyl cellulose, methyl cellulose, or the like.

In order to impart a drug component to the device of the present invention, the drug component (which may have been subjected to treatment for sustained-release dosage form) is adhered or applied to the device. For sustained-release dosage form, conventional techniques used to prepare durable preparations are adopted. For example, a sustained-release tablet is prepared and the tablet is fixed to the device.

The item to be fixed may be at a limb portion or at a centre portion (usually of shape memory material) of the device. The method of fixing may be varied depending on preparation form; in the case of, e.g. a tablet, a socket-like housing can be provided at the central part made of shape memory material, and the tablet sealed in the housing. Alternatively, a drug-containing material may be applied to a limb to form a layer by means of casting, dipping or conventional spray coating.

In one such casting method a drug is mixed with a single base or a plurality of bases of melting point higher than room temperature. The mixture is heated to dissolve or suspend the drug and the melt is immediately poured into a mold having a desired shape and a limb portion for a device of the present invention is placed in the mold. After cooling to room temperature, the cast is withdrawn. As the base, any substance can be used as long as it is a suitable additive for drugs, can adhere to the limb portion and has a melting point higher than room temperature. The base is appropriately chosen to obtain an adequate release rate, depending on the physical properties of the drug. Specific examples of the base include oils and fats, higher fatty acids, high alcohols, higher fatty acid esters and mixtures thereof. For achieving a more suitable drug release rate, a known coating may also be applied on the thus cast drug layer for release control.

In one dipping method, a drug and a base are suspended or dissolved in water or an organic solvent. The limb portion of the device of the present invention is dipped in the suspension or solution for a short period and then withdrawn. Immediately thereafter, the solvent is removed by heating or air-drying to precipitate and adhere the drug and base onto the surface of the limb. The procedures of dipping and removal of the solvent are repeated to obtain accummulated layers containing the drug on the surface of the limb. The base used is chosen from substances which can be used as additives to drugs and have the property of readily adhering to the limb portion after removal of the solvent, so as to achieve an appropriate release rate depending upon physical properties of the drug. Specific examples include substances exemplified as coating agents in the present specification. Any solvent can be used without any particular restriction, provided that it can suspend or dissolve these substances and can be removed with heating or by air-drying.

Where possible, the drug component can instead be mixed with the erodible material.

When the device of the present invention is applied clinically a feeling of satiety follows administration, and therefore the device can be used as an agent for the treatment of obesity. The present invention is not only applicable to delivery of drugs for the treatment of diseases associated with the stomach or gastrointestinal tracts such as the small intestine, colon, etc. but also to all drugs which are absorbed through the digestive tract and require duration in blood concentration.

The device according to the invention is applicable to, for example, Pirenzepine hydrochloride for the treatment of diseases associated with the digestive tract; Ambroxol, Theophylline and Ketotifen fumarate as antitussives and expectorants; Allopurinol and Benzbromarone for the treatment of gout; Disopyramide phosphate for the treatment of arrhythmia; Nicardipine hydrochloride and Nifedipine as vasodilators; Terfenadine for the treatment of allergic diseases (rhinitis, pollen allergy); Eperipson hydrochloride as antispasmodic; Tamoxifen citrate as anti-cancer agent; Bromocriptine mesilate, etc.

The devices of the present invention can be manufactured by a number of processes, for example, according to the following prcesses.
(1) The shape memory material or the portion containing it is heated, and the desired shape of the device is constructed by applying pressure followed by cooling under pressure below the Tg or the melting point. Then the or each part of in vivo erodible material, of previously fixed shape, is jointed with the shape memory material by using a suitable adhesive agent and/or by applying a mechanical interlocking method.
(2) A filamentous or ribbon-like shape memory material is sandwiched between in vivo erodible layers and a desired shape is constructed by compression molding and cutting to final form (Y-shape, cruciform, etc). If necessary, a suitable adhesive agent may be combined.
(3) A shape memory material and an erodible material are put in a metallic mold, and a desired shape is formed according to method (1). The materials may be put in the mold together or separately to give contacting surfaces between them. Between the surfaces a substance having affinity to both materials may put to join them.

For manufacturing devices according to the invention, any manufacturing equipment may be used. Manufacturing machines used are, for example, heat compression plastics molding machines, extrusion molding machines, injection molding machines, etc.

When a coating is applied to the device to control drug release, the part erodible in vivo is most usually coated. Where a mixture of the shape memory and erodible materials is used, the whole device may be coated.

In order to fold the thus prepared device, an external force is applied at a temperature higher than the m.p. or Tg of the shape memory substance to form the desired shape. Then the device is cooled to near room temperature. For easier administration, the system may also be coated with a conventionally used component or enclosed in a capsule.

The device of the present invention can be converted into a small-sized shape which is readily administered, by folding the shape memory part. This small-sized form is not maintained by constraint of elastic material so that there is no danger of the constraint being released prior to administration; however the device can revert to its original shape in the stomach after administration. The device of the present invention is of readily controlled gastric residence time, since the period for erosion of the erodible portion can be controlled by appropriate choice of erodible material and/or coating agent.

The present invention is described below in more detail by referring to the Examples.

In the Reference Examples, bending strength when the thickness of a flat plate made of shape memory substance used in the present invention is varied, and the gastric retention test of the Y-shaped and cross-shaped samples prepared from the flat plate, are explained.

### Reference Example 1

### (a) Preparation of polynorbornene flat plate and determination of bending strength of the flat plate:

After 2 g of polynorbornene was subjected to compression molding at 150°C under 100 kg/cm², the molding was cooled to room temperature to give a flat disk having a thickness of 1 mm and a diameter of 5 cm. The disk was cut into a rectangular flat plate having a thickness of 1 mm, a length of 35 mm and a width of 10 mm.

In a similar manner, rectangular flat plates having a thickness of 1.5 mm were obtained from 3 g and 4 g of polynorbornbene, respectively.

With these rectangular flat plates, the maximum load (the maximum force required to bend or break the flat plate) was determined in a water bath at 37°C in a manner similar to Testing Method for Flexural Creep of Plastics (JIS, K7116). The device used was a Rheometer NRN-2010J-CW manufactured by Fudo Co., Ltd. The test speed was 2 cm/min and the interval between supporting stands was 25 mm.

### (b) Preparation of cross-shaped samples made of polynorbornene and gastric retention test of the samples:

Flat disks having a thickness of 1 mm and a diameter of 5 cm obtained in (a) were cut to prepare cross-shaped samples having a shortest diameter of 3 cm and a width of 4 mm. Such samples were folded at 40°C. After cooling to room temperature, the samples were packed in size No.0 capsules.

In a similar manner, cross-shaped samples were prepared from flat plates having thicknesses of 1.5 mm and 2 mm, respectively.

The cross-shaped samples were administered to fasted and fed male Beagle dogs (weighing 12-16 kg). Twenty four hours after administration, a gastroendoscope was inserted to observe the content of the stomach.

The results obtained by measuring the maximum load in (a) described above and the result of the gastric retention test determined in (b) are shown below.

**TABLE 1**

| Bending strength and gastric retention of sample pieces. | | | |
|---|---|---|---|
| Thickness | Bending Strength | Gastric Rention, % (Retention Rate) | |
| | | Fasted | Fed |
| 1 mm | 252 g | 50 (3/6) * | 67 (4/6) |
| 1.5 mm | 690 g | 71 (5/7) | 100 (6/6) |
| 2 mm | 1282 g | 100 (6/6) | 100 (6/6) |

| | | | |
|---|---|---|---|
| * Numerals in parentheses indicate the number of dogs with gastric retention/the total number of dogs tested. | | | |

These results reveal that when tested under the conditions in this Reference Example, the maximum load of the rectangular plate must be 690 g or more, for retention of the device in the fed stomach for 24 hrs. or more.

### Reference Example 2

### (a) Preparation of Y-shaped samples made of polynorbornene and gastric retention test of the sample

The flat discs having a thickness of 1 mm obtained in Reference Example 1 (a) were cut to prepare Y-shaped samples having a shortest diameter of 3 cm and a width of 4 mm.

In a similar manner, Y-shaped samples were prepared from flat plates having a thickness of 1.5 mm.

With these samples, the maximum loads in two directions (vertical and horizontal, i.e. perpendicular and parallel to the plane of the samples) was determined in a water bath at 37°C using a Rheometer NRN-2010J-CW (test speed: 2cm/min; cylinder diameter: 2.5cm).

### (b) Gastric retention test of the Y-shaped sample made of polynorbornene:

The two kinds of Y-shaped sample obtained in (a) were folded at 40°C. After cooling to room temperature, the samples were packed in size No.0 capsules.

### Gastric retention test:

6 male Beagle dogs were fasted for 1 day; 30 minutes after giving a solid feed, each of the test samples was administered together with 50 ml of water. Twelve and twenty-four hours after administration, a gastroendoscope was inserted to count the number of test samples retained in the stomach.

The results are shown in the following Table 2.

**TABLE 2**

| Thickness | Strength (g) | | Gastric Retention, % (retention rate) | |
|---|---|---|---|---|
| | Vertical | horizontal | 12 hours | 24 hours |
| 1.0 mm | 104 | 280 | 83(5/6)* | 67(4/6) |
| 1.5 mm | 323 | 378 | 100(6/6) | 100(6/6) |

| | | | | |
|---|---|---|---|---|
| * Numerals in parentheses indicate the number of dogs with gastric retention/the total number of dogs tested. | | | | |

### Reference Example 3

Flat discs having a thickness of 2 mm made of polynorbornene obtained in a similar manner to Reference Example 1 (a) were cut to prepare Y-shaped samples having a shortest diameter of 3 cm and a width of 2 mm. The samples were folded at 40°C. After cooling to room temperature, the samples were packed in size No.1 capsules.

In a similar way, Y-shaped samples having a thickness of 2.5 mm, a width of 2.5 mm and a shortest diameter of 3 cm, and Y-shaped samples having a thickness of 2.8 mm, a width of 2.8 mm and a shortest diameter of 3 cm were obtained, and were packed in size No.1 capsules.

### Gastric retention test:

Gastric retention was tested for the above Y-shaped samples by the same method as Reference Example 2. The results are shown in the following Table 3.

**TABLE 3**

| Thickness | Strength (g) | | Gastric Retention, % (retention rate) | |
|---|---|---|---|---|
| | Vertical | horizontal | 12 hours | 24 hours |
| 2.0 mm | 350 | 90 | 33(2/6)* | 33(2/6) |
| 2.5 mm | 382 | 128 | 83(5/6)* | 83(5/6) |
| 2.8 mm | 886 | 250 | 100(6/6) | 100(6/6) |

| | | | | |
|---|---|---|---|---|
| * Numerals in parentheses indicate the number of dogs with gastric retention/the total number of dogs tested. | | | | |

These results (Reference Examples 2 and 3) reveal that the strength (the maximum load) of the Y-shaped sample made of polynorbornene must be 323 g (vertical direction) and 250 g (horizontal direction) respectively, for retention in the stomach for 24 hrs. or more after feeding.

### EXAMPLE 1

### (a) Preparation of polynorbornene Y-shaped part:

A polynorbornene flat disk having a thickness of 1.5 mm obtained in Reference Example 1 (a) was cut to prepare a Y-shaped sample having a shortest diameter of 1.3 cm and a width of 4 mm.

### (b) Preparation of Polyethylene Oxide 18 ^{R} flat plate part:

Using an oil press, 3.5 g of Polyethylene Oxide 18 ^{R} (PEO-18 ^{R} , manufactured by Seitetsu Kagaku Co.,Ltd.) was subjected to compression molding at a temperature of 110°C under a pressure of 100 kg/cm² to give a flat plate having a length of 1.7 cm, a thickness of 1.5 mm and a width of 4 mm.

### (c) Preparation of gastric device using two parts in combination:

Flat plates obtained in (b) were adhered one by one to the Y-shaped sample obtained in (a) above at the tip portion of each limb to prepare a gastric drug retainer as shown in Fig.1.

### EXAMPLE 2

### (a) Preparation of polynorbornene Y-shaped part:

A polynorbornee flat disk having a thickness of 1.5 mm obtained in Reference Example 1 (a) was cut to prepare a Y-shaped sample having a shortest diameter of 1.3 cm and a width of 4 mm.

### (b) Preparation of PVA-210 ^{R} flat plate part:

Using an oil press, a mixture of 3.43 g of PVA-210 ^{R} (trademark, manufactured by Kuraray Co.,Ltd., component: polyvinyl alcohol) and 0.07 g of glycerine was subjected to compression molding at a temperature of 160°C under a pressure of 100 kg/cm² to give flat plates having a length of 1.7 cm, a thickness of 1.5 mm and a width of 4 mm.

### (c) Preparation of gastric device using two parts in combination:

Flat plates obtained in (b) were adhered one by one to the Y-shaped sample obtained in (a) above at the tip portion of each limb to prepare a gastric retainer as shown in Fig.1.

### EXAMPLE 3

### (a) Preparation of polynorbornene pillar-shaped part:

A rectangular flat plate made of polynorbornene having a thickness of 2 mm obtained in Reference Example 1 (a) was cut to prepare a pillar-shaped part having a thickness of 2 mm, a width of 2.25 mm and a length of 5 mm.

### (b) Preparation of Polyethylene Oxide 18 ^{R} pillar-shaped part:

Using an oil press, 4 g of Polyethylene Oxide 18 ^{R} was subjected to compression molding at a temperature of 110°C under a pressure of 100 kg/cm² to give a pillar-shaped part having a length of 1.5 cm, a thickness of 2 mm and a width of 2.25 mm.

### (c) Preparation of gastric device:

The pillar-shaped part obtained in (a) above was perpendicularly adhered to the Y-shaped gastric device obtained in Exmaple 1 at the central portion thereof by epoxy resin and a part obtained in (b) above was further adhered thereon at the tip portion thereof by epoxy resin to prepare a gastric device as shown in Fig.3(c).

### EXAMPLE 4

The gastric device obtained in Example 1 was folded at 40°C and then cooled to room temperature. A 6% aqueous solution of hydroxypropylmethyl cellulose was coated onto the folded device at 30°C in a conventional manner to give a gastric device for oral administration in accordance with the present invention.

### EXAMPLE 5

### (a) Preparation of coating solution:

In methanol were dissolved 4 parts of EUDRAGIT RS and 1 part of EUDRAGIT L100 to make a 10% (wt/wt) solution. As plasticizer, 2% (wt/wt) of triacetin was added to the solution to prepare a coating solution.

### (b) Preparation of coated device:

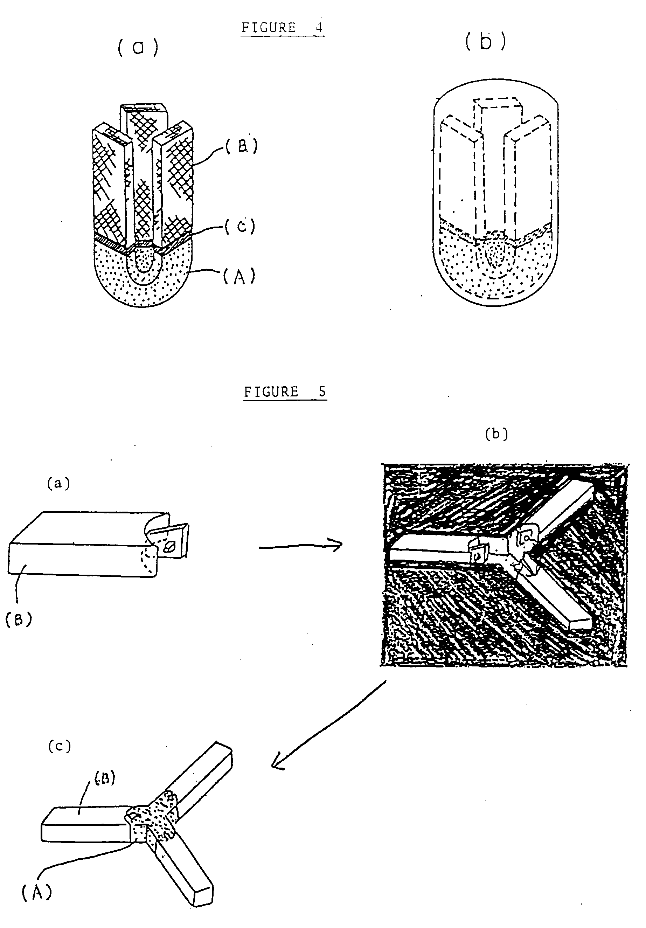

The coating solution obtained above was coated onto the Polyethylene Oxide 18 ^{R} parts of Y-shaped gastric drug retainers obtained in Example 1. The amounts of coating were 20%, 25%, 30% and 35% (wt/wt), based on the total weight. The thus obtained retainers were folded at 40°C. After cooling to room temperature (Fig.4 (a)), each retainer was encased in a No.0 capsule (Fig.4 (b)).

### Gastric retention test:

Gastric retention was examined with the gastric drug retainers described above according to the method described in Reference Example 2. The results are shown in Table 4.

**TABLE 4**

| Coating Rate | Gastric Retention, % (retention rate) | |
|---|---|---|
| | 12 Hours | 24 Hours |
| 20% | 67 (4/6) * | 33 (2/6) |
| 25% | 67 (4/6) | 17 (1/6) |
| 30% | 50 (3/6) | 17 (1/6) |
| 35% | 100 (6/6) | 83 (5/6) |

| | | |
|---|---|---|
| * Numerals in parentheses indicate the number of dogs with gastric retention.the total number of dogs tested. | | |

### EXAMPLE 6

### Preparation of polynorbornene Y-shaped part:

Polynorbornene flat disks having a thickness of 2.8 mm obtained in Reference Example 3 were cut to prepare Y-shaped samples having a shortest diameter of 1.3 cm and a width of 2.8 mm.

### (b) Preparation of polyethylene oxide 18 flat plate part:

Using an oil press, 6.0 g of polyethylene oxide 18 (PEO-18, manufactured by Seitetsu Kagaku Kogyo Co.,Ltd.) was subjected to compression molding at a temperature of 110°C under a pressure of 100 kg/cm² to give a flat disc having a thickness of 2.8 mm. The flat discs were cut to prepare flat plates having a length of 1.7 cm, a thickness of 2.8 mm and a width of 2.8 mm.

### (c) Preparation of gastric device using two parts in combination:

The first plates obtained in (b) were adhered one by one to the Y-shaped samples obtained in (a) above at the tip portion of each limb using epoxy resin adhesive to prepare gastric drug retainers as shown in Fig.1.

### (d) Preparation of coated gastric drug retainer and its gastric retention test:

The coating solution obtained in Example 5 was coated onto the Polyethylene Oxide 18 parts of the gastric drug retainers in amounts of 5%, 10% and 15% (wt/wt), based on the total weight. The thus obtained retainers were folded at 40°C. After cooling to room temperature (Fig.4 (a)), each retainer was encased in a size No.0 capsule (Fig.4 (b)).

### Gastric Retention test:

Gastric retention was examined with the gastric drug retainers described above according to the method described in Reference Example 2. The results are shown in Table 5.

**TABLE 5**

| Coating Rate | Gastric Retention % (Retention Rate) | |
|---|---|---|
| | 12 Hours | 24 Hours |
| 5% | 0 (0/6)* | -- |
| 10% | 67 (4/6) | 17 (1/6) |
| 15% | 67 (4/6) | 17 (1/6) |

| | | |
|---|---|---|
| * Numerals in parentheses indicate the number of dogs with gastric retention/the total number of dogs tested. | | |

### EXAMPLE 7

### (a) Preparation of polynorbornene Y-shaped part:

Polynorbornene flat discs having a thickness of 2.8 mm. obtained in Reference Example 3 were cut to prepare Y-shaped samples having a shortest diameter of 1.3 cm and a width of 2.8 mm.

### (b) Preparation of polyvinylalcohol flat plate part:

Using an oil press, mixtures of Denkapoval B24 (trade name, manufactured by Denki Kagaku Kogyo K.K., component: partially saponified polyvinylalcohol) and glycerine were subjected to compression molding under the conditions of Example 2b) to give flat discs having a thickness of 2.8 mm. The ratios of polyvinylalcohol to glycerine (w/w %) are 5% 15% and 23% respectively. These flat discs were cut to prepare flat plates having a length of 1.7 cm and a width of 2.8 mm.

### (c) Preparation of gastric drug retainers using two parts in combination:

Flat plates obtained in (b) were adhered one by one to the Y-shaped samples obtained in (a) above at the tip portion of each limb by using epoxy resin adhesive to prepare gastric drug retainers as shown in Fig.1.

### (d) Preparation of coated gastric drug retainer and its gastric retention test:

The coating solution obtained in Example 5 was coated onto the polyvinylalcohol parts of the gastric drug retainers in amounts of 5%, 7.5%, 10% and 15% (wt/wt), based on the total weight. The thus obtained retainers were folded at 40°C. After cooling to room temperature (Fig.4 (a)), each retainer was encased in a size No.0 capsule (Fig.4 (b)).

### Gastric Retention Test:

Gastric retention was examined with the gastric drug retainers described above according to the method described in Reference Example 2. The results are shown in Table 6.

**TABLE 6**

| Glycerine % | Coating % | Gastric Retention, % (retention rate) | |
|---|---|---|---|
| | | 12 hours | 24 hours |
| | 7.5 | 67 (4/6) * | 0 (0/6) |
| 5 | 10.0 | 83 (5/6 | 50 (3/6) |
| 15 | 7.5 | 20 (1/5) | 0 (0/5) |
| 23 | 7.5 | 50 (3/6) | 0 (0/6 |
| | 10.0 | 60 (3/5) | 20 (1/5) |

| | | | |
|---|---|---|---|
| * Numerals in parentheses indicate the number of dogs with gastric retention/the total number of dogs tested. | | | |

### EXAMPLE 8

### (a) Preparation of PEO-18 pillar part having a junction protrusion:

Using an injection molding machine (40/15MSS, manufactured by Mitsubishi Heavy Industries, Ltd.), PEO-18 was subjected to injection molding under the conditions shown below to give a pillar part having a length of 1.4 cm, a thickness of 2.8 mm and a width of 2.8 mm, and having a junction protrusion (Fig.5 (a)).
Conditions of the injection molding:

| Temperature (°C) | | | | Injection Pressure (Kg/cm²) | Injection Speed | Metal Mold Temp (°C) |
|---|---|---|---|---|---|---|
| Cylinder | | | Nozzle | | | |
| Rear | Middle | Front | | | | |
| 140 | 145 | 150 | 150 | 1600-1800 | Slow | 25 |

### (b) Preparation of gastric drug retainer using injection molding (insert-molding):

Three pillar parts obtained in (a) were inserted into a metal mold for injection molding at intervals of 120° radially. For the centre portion among these parts, the shape memory substance, Diary MM-4500 (trade name, manufactured by Mitsubushi Heavy Industries, Ltd., component: polyurethane) was subjected to injection molding to join the pillar parts (obtained in (a)) to it to give a gastric drug retainer having a shortest diameter of 3 cm, a thickness of 2.8 mm and a width of 2.8 mm (Fig. 5 (c)).
Conditions of the injection molding:

| Temperature (°C) | | | | Injection Pressure (Kg/cm²) | Injection Speed | Metal Mold Temp (°C) |
|---|---|---|---|---|---|---|
| Cylinder | | | Nozzle | | | |
| Rear | Middle | Front | | | | |
| 195 | 205 | 215 | 200 | 800-1200 | Fast | 25 |

Strength of the gastric drug retainer described above in vitro was examined in a manner similar to the test performed in Reference Example 2. The results are shown in Table 7.

### EXAMPLE 9

### (a) Preparation of PVA B-24 pillar part having a junction protrusion:

To PVA B-24 (trade name, made by Denki Kagaku Kogyo K.K., component: partially saponified polyvinyl alcohol) was added glycerine (as a plasticizer) in the ratio of 10% (w/w), and the resultant was mixed using a grinding machine. The resultant mixture was dried for 2 hours at 105°C, and subjected to injection molding to give a pillar part having a length of 1.4 cm, a thickness of 2.8 mm and a width of 2.8 mm, and having a junction protrusion.
Conditions of the injection molding:

| Temperature (°C) | | | | Injection Pressure (Kg/cm²) | Injection Speed | Metal Mold Temp (°C) |
|---|---|---|---|---|---|---|
| Cylinder | | | Nozzle | | | |
| Rear | Middle | Front | | | | |
| 155 | 195 | 215 | 200 | 1600-1800 | Slow | 65 |

### (b) Preparation of gastric drug retainer using injection molding (insert-molding):

Three pillar parts obtained in (a) were inserted into a metal mold for injection molding at intervals of 120° radially. For the centre portion among these parts, Diary (the shape memory substance) was subjected to injection molding to join the pillar parts (obtained in (a)) to it to give a gastric drug retainer having a shortest diameter of 3 cm, a thickness of 2.8 mm and a width of 2.8 mm. Strength of the device in vitro was examined in a manner similar to the test of Reference Example 2. The results are shown in Table 7.

### EXAMPLE 10

### (a) Preparation of HPC-H pillar part having a junction protrusion:

HPC-H (trade name, made by Nippon Soda Co.,Ltd., component: hydroxypropylcellulose) was subjected to injection molding to give a pillar part having a length of 1.4 cm., a thickness of 2.8 mm and a width of 2.8 mm, and having a junction protrustion.
Conditions of the injection molding:

| Temperature (°C) | | | | Injection Pressure (Kg/cm²) | Injection Speed | Metal Mold Temp (°C) |
|---|---|---|---|---|---|---|
| Cylinder | | | Nozzle | | | |
| Rear | Middle | Front | | | | |
| 140 | 150 | 160 | 150 | 800-1200 | Slow | 65 |

### (b) Preparation of gastric device using injection molding (insert-molding):

Three pillar parts obtained in (a) were inserted into a metal mold for injection molding at intervals of 120° radially. For the centre portion among these parts, Diary was subjected to injection molding to join the pillar parts (obtained in (a)), to it to give a gastric device having a shortest diameter of 3 cm, a thickness of 2.8 mm and a width of 2.8 mm. Strength of the gastric device described above in vitro was examined in a manner similar to the test performed in Reference Example 2. The results are shown in Table 7.

### EXAMPLE 11

### (a) Preparation of HPC-H/Ethocel mixture pillar part having junction protrusion:

To HPC-H (trade name, made by Nippon Soda Co.,Ltd., component: hydroxypropylcellulose) were added Ethocel STD4 (trade name, made by Dow Chemicals Ltd., component: ethylcellulose) in the ratio of 20% (w/w) and PEG 400 in the ratio of 15% (w/w), and the resultant was mixed using a grinding mixing machine. The resultant mixture was dried at 105°C for 2 hours, and subjected to injection molding under the conditions mentioned in Example 10 (a) to give a pillar part having a length of 1.4 cm, a thickness of 2.8 mm and a width of 2.8 mm and having a junction protrustion.

### (b) Preparation of gastric device using injection molding (insert-molding):

Three pillar parts obtained in (a) were inserted into a metal mold for injection molding at intervals of 120° radially. For the centre portion among these parts, Diary was subjected to injection molding to join the pillar parts (obtained in (a)) to it to give a gastric retention device having a shortest diameter of 3 cm, a thickness of 2.8 mm and a width of 2.8 mm. Strength of the gastric device described above in vitro was examined in a manner similar to the test performed in Reference Example 2. The results are shown in Table 7.

**TABLE 7**

| Test results for strength of gastric devices in vitro. | | |
|---|---|---|
| Gastric Device | Strength (g) | |
| | Vertical | Horizontal |
| Ex.8 | 848 | 322 |
| Ex.9 | 348 | 155 |
| Ex.10 | 649 | 272 |
| Ex.11 | 339 | 155 |

### EXAMPLE 12

### (a) Preparation of polynorbornene Y-shaped part (Part A) with junction protrusion:

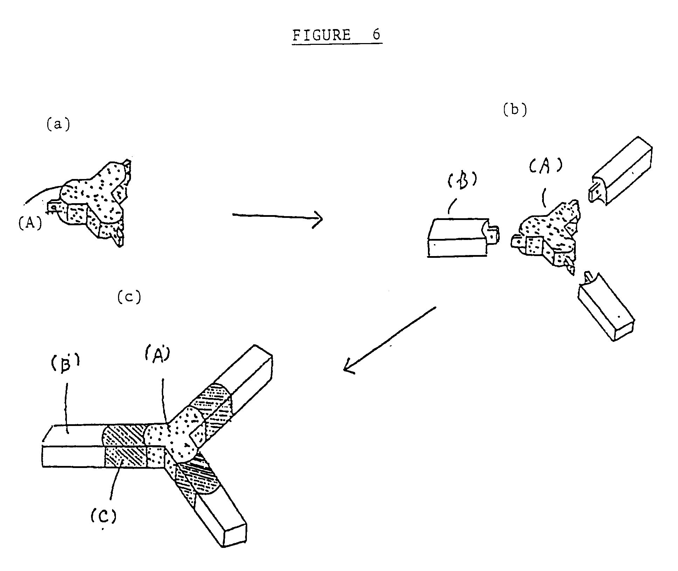

5.2 g of polynorbornene was subjected to compression molding at 150°C and at a pressure of 100 kg/cm², and cooled to room temperature to give a plate disc having a thickness of 2.8 mm and a diameter of 5 cm. These plate discs were cut to prepare Y-shaped parts having a shortest diameter of 1 cm, a width of 2.8 mm, and a junction protrusion (Part A) (Fig.6 (a)).

### (b) Preparation of PEO-18 pillar part having junction protrusion (Part B)

PEO-18 was subjected to injection molding in a manner similar to Example 8 (a) to give a pillar part having a length of 1.1 cm, a thickness of 2.8 mm and a width of 2.8 mm (Part B).

### (c) Preparation of gastric device obtained by combining the two kinds of parts using injection molding of polyurethane:

A centre portion A (obtained in (a) above) and three Parts B at radial intervals of 120° were inserted in a metal mold for injection molding as shown in Fig.6 (b). Between Parts A and B was inserted thermoplastic polyurethane (trade name: Mirakutoran E280, made by Nihon Miracutoran Co.,Ltd.,) by injection molding under the conditions shown below to join parts A and B to give a gastric retention device (Fig.6 (c)).
Conditions of the injection molding:

| Temperature (°C) | | | | Injection Pressure (Kg/cm²) | Injection Speed | Metal Mold Temp (°C) |
|---|---|---|---|---|---|---|
| Cylinder | | | Nozzle | | | |
| Rear | Middle | Front | | | | |
| 160 | 180 | 190 | 200 | 800-1200 | Slow | 35 |

### EXAMPLE 13

A centre portion A obtained in Example 12 (a), and three Parts B obtained in Example 12 (b) at radial intervals of 120° were inserted in a metal mold for injection molding as shown in Fig.6 (b). Between Parts A nad B Diary MM-4500 (shape memory substance) was inserted and subjected to injection molding under the conditions of Example 8 (b) to join Parts A and B to give a gastric device (Fig.6 (c)).

### EXAMPLE 14

### (a) Preparation of polynorbornene flat plate (Part A):

Using an oil press, 1 g of polynorbornene was subjected to compression molding at 150°C under 100 kg/cm² to give a flat disk having a thickness of 0.5 mm and a diameter of 5 cm. The disk was cut into widths of 2 mm with an interval of 1 mm (Part A).

### (b) Preparation of Polyethylene Oxide 18 ^{R} flat plate (Part B):

Using an oil press, 1 g of Polyethylene Oxide 18 ^{R} was subjected to compression molding at a temperature of 110°C under a pressure of 100 kg/cm² to give a flat disk having a thickness of 0.5 mm and a diameter of 5 mm.

### (c) Preparation of gastric drug retainer using two parts in combination:

Two Parts A obtained in (a) above were inserted between three Parts B obtained in (b) above, with the cut widths of parts A aligned as in Fig.7 (a). After the resulting parts were overlaid to true up the circumferences, the layered parts were subjected to compression molding at 110°C under 100 kg/cm² and the resulting molding was cooled to room temperature to give a flat disk having a thickness of 2 mm and a diameter of 5 cm. The flat disk was cut to prepare a cross-shaped sample having a shortest diameter of 3 cm and a Y-shaped sample (cf.Fig.7 (b)). The samples were folded at 40°C (cf.Fig.7 (c)). After cooling to room temperature, each sample was encased in a No.00 capsule.

Shape restorability and erodibility of the gastric drug retainers described above in vitro were examined using a Sartorius elution tester (100 ml of JP XI disintegration test medium I, 160 g of beads, 37°C, 1.2 rpm). The test results are shown in Table 8.

**TABLE 8**

| Shape restorability and erodibility of gastric drug retainer in vitro. | | | |
|---|---|---|---|
| Item Tested | 1 Hour | 12 Hours | 24 Hours |
| Shape restorability | Completely restored | | |
| Erodibility | Little changed | Water permeated to swell | Eroded into small pieces of 4-5 mm |

### EXAMPLE 15

### (a) Preparation of PVA-210 ^{R} flat plate:

Using an oil press, a mixture of 0.85 g of PVA-210 ^{R} and 0.15 g of glycerine was subjected to compression molding at a temperature of 160°C under a pressure of 100 kg/cm² to give a flat disk having a thickness of 0.5 mm and a diameter of 5 cm (Part D)

### (b) Preparation of gastric drug retainer using two parts in combination:

Two parts obtained in Example 14 (a) were inserted between three parts obtained in (a) above, each part being inserted as in Example 14 and Fig. 7(a). After the resulting part composites were overlaid to true up the circumferences, the layered parts were subjected to compression molding at 130°C under 100 kg/cm² and the resulting molding was cooled to room temperature to give a flat disk having a thickness of 2 mm and a diameter of 5 cm. The flat disk was cut to prepare a cross-shaped sample having a shortest diameter of 3 cm and a Y-shaped sample. The samples were folded at 40°C. After cooling to room temperature, each sample was encased in a No.00 capsule to prepare a gastric drug retainer.

Shape restorability and erodibility of the gastric drug retainers described above in vitro were examined by the rotary flask method (40 ml of JP XI disintegration test medium I, 150 g of beads, 37°C, 40 rpm). The test results are shown in Table 9.

**TABLE 9**

| Shape restorability and erodibility of gastric drug retainer in vitro. | | | |
|---|---|---|---|
| Item Tested | 1 Hour | 12 Hours | 24 Hours |
| Shape restorability | Completely restored | | |
| Erodibility | Little changed | Water permeated to swell | Eroded into small pieces of 4-5 mm |

### EXAMPLE 16

Using an oil press, 4 g of a mixture obtained by formulating 1 to 4 parts of PEO-18 ^{R} in 1 part of polynorbornene was subjected to compression molding to give a flat plate having a thickness of 2 mm and a diameter of 5 cm. This flat plate was cut into a cross-shaped sample having a shortest diameter of 3 cm and a width of 4 mm. The thus obtained retainer was folded at 40°C. After cooling to room temperature, each retainer was encased in a No.000 capsule to prepare a gastric drug retainer.

Shape restorability and erodibility of the gastric drug retainers described above in vitro were examined in a manner similar to the test performed in Example 14. The test results are shown in Table 10.

**TABLE 10**

| Polynorborne:PEO-18 ^{R} | Shape Restorability (30 mins). | Erodibility (24 hours) |
|---|---|---|
| 50 : 50 | Completely restored | No erosion |
| 40 : 60 | Completely restored | No erosion |
| 35 : 65 | Completely restored | No erosion |
| 30 : 70 | Completely restored | No erosion |
| 25 : 75 | Completely restored | No erosion |
| 20 : 80 | Completely restored | Eroded |

### EXAMPLE 17

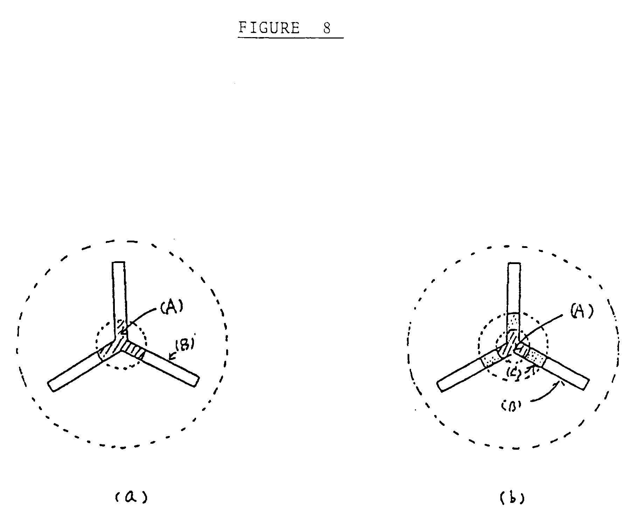

0.3 g of polynorbornene and 6.88 g of PVA B-24 (containing glycerine as plasticizer) were placed successively as coaxial circles with radii being 6 mm and 25 mm, and were subjected to compression molding at 130°C under 100 kg/cm². The molding was cooled to room temperature to give a flat disk having a thickness of 2.8 mm and a diameter of 5 cm. The disk was cut to prepare a Y-shaped sample having a shortest diameter of 3 cm and a width of 2.8 mm (Fig.8 (a)).

Strength of the sample (the gastric device) described above in vitro was examined according to the method shown in Reference Example 2. The results are shown in Table 11.

### EXAMPLE 18

0.18 g of polynorbornene, 0.43 g of mixed equal amounts of polynorbornene and PEO-18, and 5.38 g of PEO-18 were placed successively as coaxial circles with radii being 4 mm, 8 mm and 25 mm, and were subjected to compression molding at 130°C under 100 kg/cm². The molding was cooled to room temperature to give a plate disk having a thickness of 2.8 mm and a diameter of 5 cm. The disk was cut to prepare a Y-shaped gastric device having a shortest diameter of 3 cm and a width of 2.8 mm.

Strength of the gastric device in vitro was examined according to the method shown in Reference Example 2. The results are shown in Table 11.

### EXAMPLE 19

0.18 g of polynorbornene, 0.43 g of Diary (MM-4500) and 5.38 g of PEO-18 were placed successively as coaxial circles with radii being 4 mm, 8 mm and 25 mm, and were subjected to compression molding at 130°C under 100 kg/cm². The molding was cooled to room temperature to give a flat disk having a thickness of 2.8 mm and a diameter of 5 cm. The disk was cut to prepare a Y-shaped gastric device having a shortest diameter of 3 cm and a width of 2.8 mm (Fig.8 (b)).

Strength of the gastric retention device in vitro was examined according to the method shown in Reference Example 2. The results are shown in Table 11.

### EXAMPLE 20

0.18 g of polynorbornene, 0.43 g of ethylene-vinylacetate copolymer (trade name: Flovac, vinylacetate: 20%, made by Seitetsu Kagaku Co.,Ltd) and 5.38 g of PEO-18 were placed successively as coaxial circles with radii being 4 mm, 8 mm and 25 mm, and were subjected to compression molding at 130°C under 100 kg/cm². The molding was cooled to room temperature to give a flat disk having a thickness of 2.8 mm and a diameter of 5 cm. The disk was cut to prepare a Y-shaped gastric device having a shortest diameter of 3 cm and a width of 2.8 mm.

Strength of the gastric retention device in vitro was examined according to the method shown in Reference Example 2. The results are shown in Table 11.

**TABLE 11**

| Strength ofgastric devices in vitro. | | |
|---|---|---|
| Gastric Device | Strength (g) | |
| | Vertical | Horizontal |
| Ex.17 | 743 | 200 |
| Ex.18 | 353 | 96 |
| Ex.19 | 401 | 118 |
| Ex.20 | 355 | 105 |

### EXAMPLE 21

### (a) Preparation of conventional tablets

Tablets are prepared using the following formulation (active ingredient: nicardipine hydrochloride).

| | |
|---|---|
| Nicardipine hydrochloride | 40 mg. |
| Lactose | 140 mg. |
| CMC-Ca | 20 mg. |
| | 200 mg. |

### (b) Preparation of sustained-release tablets:

Tablets were prepared using the following formulation (active ingredient: nicardipine hydrochloride) by wet granulation:

| | |
|---|---|
| Nicardipine-HCl | 60 mg. |
| HPC-H | 48 mg. |
| HPC-M | 12 mg. |
| | 120 mg. |

### (c) Preparation of a gastric device to which sustained-release tablets are attached:

To the gastric retention device (Y-shaped, 25% coating) made by Example 5 were fixed the above sustained-release tablets (two, three or four) by using an epoxy resin adhesive.

### (d) Drug administration test:

One tablet obtained in (a) and a gastric device from (c), were administered to together six male Beagle dogs, together with 50 ml of water. As controls, one tablet obtained in (a) and four sustained-release tablets obtained in (b) or conventional tablets (160 mg as nicardipine hydrochloride) were administered orally similarly. The Beagle dogs were fasted for one day before the administration, and, 30 minutes before the administration, were given a solid feed (50 g); 1, 2, 4, 6, 8, 10, 12 and 24 hours after the administration, blood was taken from a fore-limb.

Plasma level of nicardipine was measured by liquid chromatography (S.Kobayashi, Journal of Chromatography, 420, 439-444, 1987).

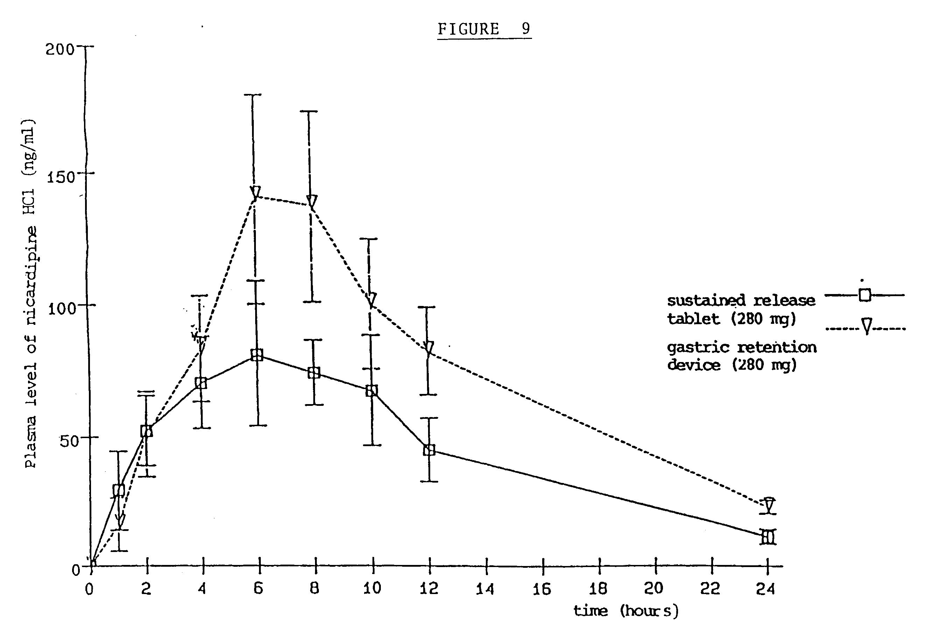

In Fig.9 are shown the changes of plasma level in the case of four sustained-release tablets attached to a gastric device and one conventional tablet administered at the same time and in the case of four sustained-release tablets and one conventional tablet being administered at the same time. As a result, it is apparent that AUC increase and the plasma level become significantly high 12 hours and 24 hours after administration when using the invention.

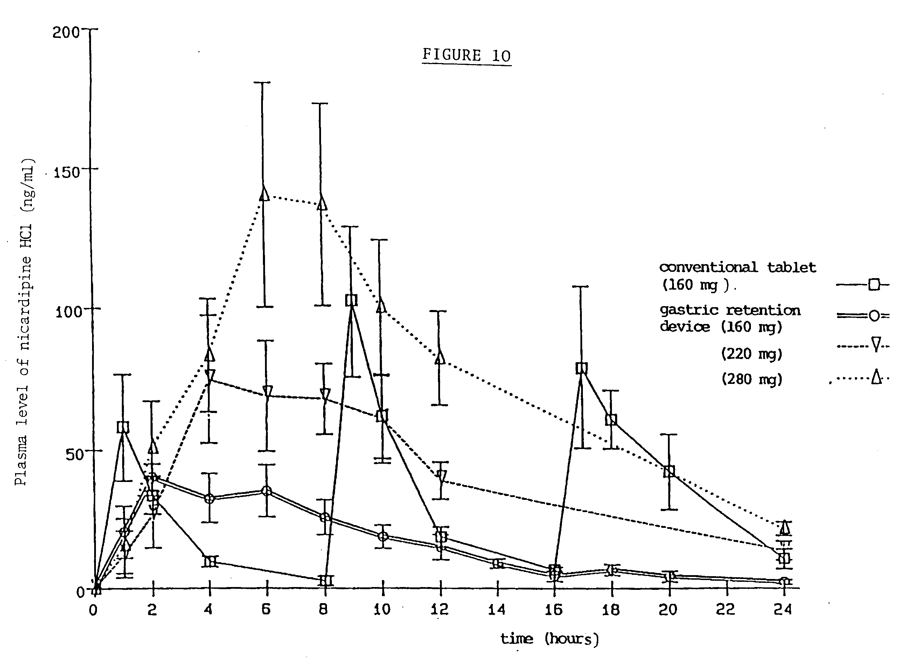

In Fig.10 are shown the changes of plasma level in the cases of respective gastric devices to which two, three and four sustained-release tablets were fixed being administered together with one tablet, and in the case of a conventional tablet (160 mg) being administered. As a result, it is apparent that, by controlling the administration amount of sustained-release tablets, good sustained release (single dose per day) can be obtained.

### EXAMPLE 22

### (a) Preparation of sustained-release layers by the casting method:

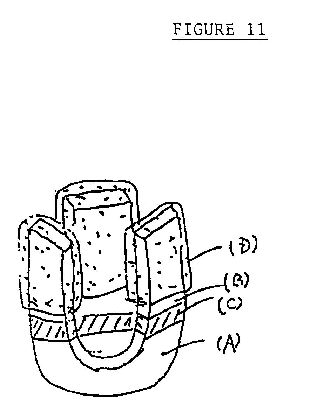

The following formulation was melted, mixed and solidified under cooling around the limb portion of the 10% coated Y-shaped gastric device obtained in Example 6 (Fig.11).
Formulation:

| | |
|---|---|
| Nicardipine hydrochloride | 60 mg. |
| Cetyl alcohol | 60 mg. |
| Polyethylene glycol 6000 | 30 mg. |
| Polyethylene glycol 400 | 30 mg. |
| | 180 mg. |

### (b) Drug administration test:

One conventional tablet obtained in Example 21 (a) and the gastric device obtained in above (a) were administered, at the same time, to six male Beagle dogs, together with 50 ml of water. The test method is similar to that of Example 21 (d).

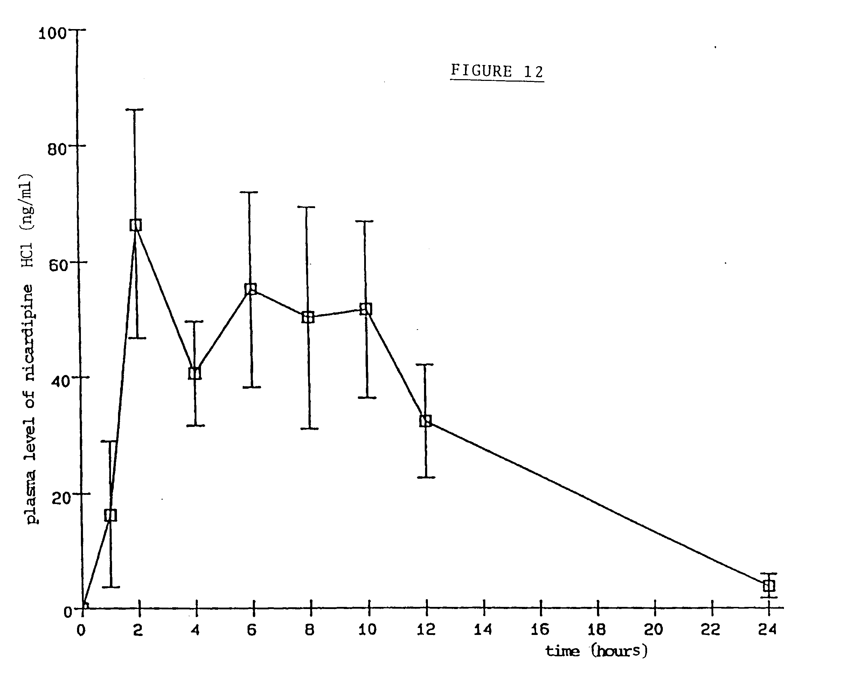

In Fig.12 is shown the change of the plasma level with time. As a result, it is apparent that, in the case of the drug administered by the casting method, good sustained-release (single dose per day) can be obtained.

### EXAMPLE 23

### (a) Preparation of drug-containing plate parts:

3 g of polynorbornene and 3 g of nicardipine hydrochloride were mixed, and subjected to compression molding using an oil press at 110°C under 100 kg/cm², and then cut to prepare flat plates having a length of 1.7 cm, a thickness of 2.8 mm and a width of 2.8 mm.

### (b) Preparation of gastric device:

The flat plates obtained in (a) were adhered by using an epoxy resin adhesive, one by one, to the Y-shaped sample obtained in Example 6 (a) at the tip portions of each limb to prepare a gastric device.

### (c) Preparation of a coated gastric device:

The drug-containing portion of the Y-shaped gastric device obtained in (b) was coated with a 5% (wt/wt of the total amount) layer using a coating solution.

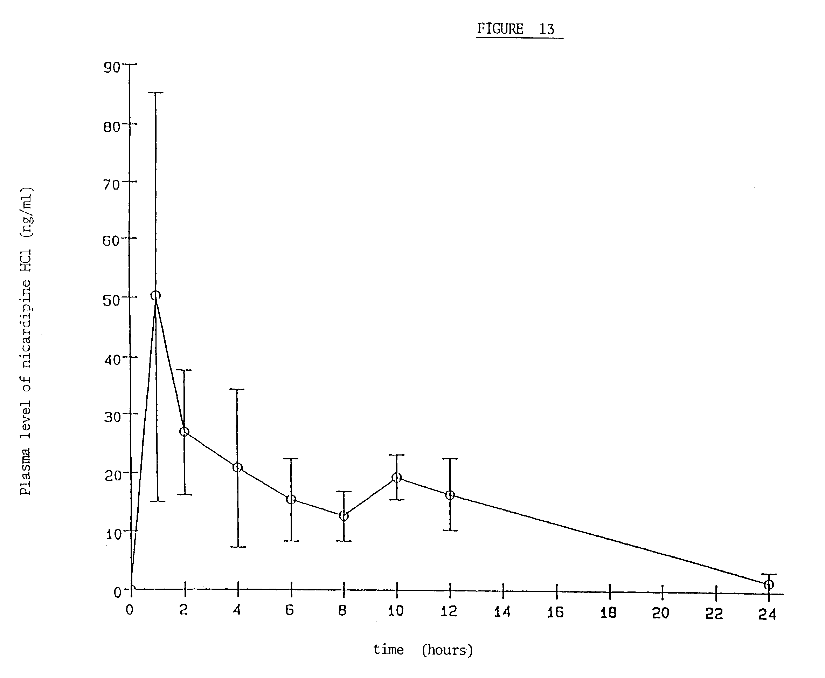

The above gastric device was administered to Beagle dogs as in Example 21, and the plasma level was measured. The results are shown in Fig.13. It is apparent that good sustained-release (single dose per day) is obtained in this case using a mixture of drug and erodible material.

A preferred device according to the invention comprises a shaped body of shape memory material and material erodible in vivo (gastrically) joined or mixed together, the shaped body being capable of being and remaining folded at a region or regions of shape memory material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach.

The shaped body may comprise a laminate of layers of the erodible material about a layer of the shape memory material which on its own can exit from the stomach - e.g. which is of filament(s), mesh strip(s), ribbon(s) or islands of the shape memory material which, when free of the erodible material, can pass from the stomach and through the patient's body. It may be of a mixture of the shape memory and erodible materials. It may instead be made of separate parts of the shape memory and erodible materials jointed together by physical engagement and/or by adhesive and/or by jointing material. A given device may employ one or more of these arrangements. The device may carry a drug (e.g. as a tablet or in a coating), preferably in sustained release form. All or part (e.g. the portion(s) of erodible material) may be coated to help control rate of erosion and/or drug release.

## Claims

1. A device for extended retention in the stomach comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach.

2. A device according to claim 1, having at least three coplanar limbs extending from a centre, the device at and/or adjacent to the centre where the limbs join being constructed with said shape memory material and the outboard portions of the limbs being constructed with said gastrically erodible material in vivo.

3. A device according to claim 1, comprising at least three coplanar limbs extending from the centre, each limb being constructed with said shape memory material disposed lattice-like between layers of said gastrically erodible material.

4. A device according to any preceding claim, wherein (a) said shape memory substance is polynorbornene or polyurethane, and/or (b) said gastrically erodible material is polyethylene oxide or polyvinyl alcohol.

5. A device according to any preceding claim, which is partially or wholly coated with substance sparingly soluble or insoluble in gastric juice.

6. A device according to claim 5, wherein said coating substance is at least one material selected from 1 : 2 : 0.1 copolymer of ethyl acrylate, methyl methacrylate and trimethyl-ammonioethyl methacrylate chloride; 1 : 1 copolymer of methyl methacrylate and methacrylic acid; 1 : 1 copolymer of ethyl acrylate and methacrylic acid; 2 : 1 copolymer of methyl methacrylate and methacrylic acid; hydroxypropylmethyl cellulose phthalate; cellulose acetate phthalate; and ethyl cellulose (most preferably with 0 to 6 : 1 mixture of 1 : 2 : 0.1 copolymer of ethyl acrylate, methyl methacrylate and trimethylammonioethyl methacrylate chloride and 1 : 1 copolymer of methyl methacrylate and methacrylic acid, in a ratio of 5 to 40 w/w%.

7. A device according to any preceding claim, which is folded to orally administrable form without constraint of elastic material.

8. A device according to any preceding claim, carrying a drug.

9. A device according to claim 8, having one or more of the following features :-
(a) the drug is nicardipine;
(b) the drug is in sustained release formulation;
(c) the drug is in tablet or thin layer form.

10. A method of making a device for extended retention in the stomach, said device comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach, which comprises forming a shape memory body comprising said shape memory material, juxtaposing the shape memory body with one or more bodies of said gastrically erodible material and joining all of the bodies together with adhesive or jointing material and/or mechanical engagement.

11. A method of making a device for extended retention in the stomach, said device comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach, which comprises molding an integral disc or plate having radially successive regions of said shape memory material and said gastrically erodible material, optionally with jointing material therebetween, and cutting the disk or plate to form a device having a plurality of arms extending through said regions.

12. A method of making a device for extended retention in the stomach, said device comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach which comprises blending said shape memory material and said gastrically erodible material and molding, or molding and cutting, the blend to form a shape memory body of the required configuration.

13. A method of making a device for extended retention in the stomach, said device comprising a shaped body of shape memory material and gastrically erodible material joined or mixed together, the shaped body being capable of being and remaining folded at a region or regions of shape memory material without constraint of elastic material to facilitate administration and of reverting in the stomach to the memorized unfolded configuration in which it is of such size, shape and durability as to be retained in the stomach without blocking the passage of food therein, and the erodible material eroding controlledly in the stomach to leave device remains which can pass from the stomach which comprises sandwiching between layers of said gastrically erodible material a discontinuous layer of said shape memory material which on its own can exit from the stomach, molding the assembly to form a composite disk or plate, and cutting the shape memory device therefrom.

14. A method according to claim 13, wherein :-
(a) said discontinuous layer is in the form of separate strips and/or threads and/or islands, and/or
(b) two or more such layers of shape memory material are sandwiched individually between correspondingly three or more such erodible layers.

15. A method according to any of claims 10 to 14 wherein (a) said shape memory substance is polynorbornene or polyurethane, and/or (b) said gastrically erodible material is polyethylene oxide or polyvinyl alcohol.

16. A method according to any of claims 10 to 15 which includes wholly or partially coating the device with substance sparingly soluble or insoluble in gastric juice.

17. A method according to any of claims 10 to 16 which includes folding the device to orally administrable form without constraint of elastic material.

18. A method according to any of claims 10 to 17 which includes applying a drug to the device or incorporating a drug in the erodible material.

19. A method according to claim 18 wherein one or more of the following features applies :
(a) the drug is nicardipine;
(b) the drug is in sustained release formulation;
(c) the drug is in tablet or thin layer form.

## Patentansprüche

1. Vorrichtung für eine zeitlich ausgedehnte Retention im Magen, die einen geformten Körper aus einem Material mit Gedächtniseffekt und einem im Magen erodierbaren Material, die miteinander verbunden oder vermischt sind, umfaßt, wobei der geformte Körper gefaltet sein und bleiben kann bei einem Bereich oder Beichen aus Material mit Gedächtniseffekt ohne Beschränkungen, die ein elastisches Material aufweist, um eine Verabreichung zu vereinfachen, und wobei sich der geformte Körper im Magen in die gespeicherte ungefaltete Anordnung zurückfalten kann, in der der geformte Körper eine solche Größe, Gestalt und Dauerhaftigkeit aufweist, daß er im Magen zurückgehalten wird, ohne in diese das Durchtreten von Nahrung zu blockieren, und wobei das erodierbare Material kontrolliert im Magen erodiert, um Vorrichtungsrückstände zu hinterlassen, die den Magen verlassen können.

2. Vorrichtung nach Anspruch 1, die mindestens drei koplanare Schenkel aufweist, die sich von einem Mittelpunkt erstrecken, wobei die Vorrichtung bei dem oder angrenzend an den Mittelpunkt, an dem die Schenkel zusammenlaufen, unter Verwendung des Materials mit Gedächtniseffekt hergestellt ist und die sich nach außen erstreckenden Bereiche der Schenkel unter Verwendung des in vivo im Magen erodierbaren Materials hergestellt sind.

3. Vorrichtung nach Anspruch 1, die mindestens drei koplanare Schenkel umfaßt, die sich von dem Mittelpunkt erstrecken, wobei jeder Schenkel unter Verwendung des Materials mit Gedächtniseffekt, das gitterähnlich zwischen Schichten des im Magen erodierbaren Materials angeordnet ist, hergestellt ist.

4. Vorrichtung nach irgendeinem der vorangegangenen Ansprüche, in der (a) die Substanz mit Gedächtniseffekt Polynorbornen oder Polyurethan und/oder (b) das im Magen erodierbare Material Polyethylenoxid oder Polyvinylalkohol ist.

5. Vorrichtung nach irgendeine der vorangegangenen Ansprüche, die teilweise oder vollständig mit einer im Magensaft nur geringfügig löslichen oder unlöslichen Substanz überzogen ist.

6. Vorrichtung nach Anspruch 5, in der die Überzugssubstanz mindestens ein Material umfaßt, ausgewählt aus eine Copolymer im Verhältnis 1:2:0,1 von Ethylacrylat, Methylmethacrylat und Trimethylammoniumethylmethacrylatchlorid, eine Copolymer im Verhältnis 1:1 von Methylmethacrylat und Methacrylsäure, eine Copolymer im Verhältnis 1:1 von Ethylacrylat und Methacrylsäure, einem Copolymer im Verhältnis 2:1 von Methylmethacrylat und Methacrylsäure; Hydroxypropylmethylcellulosephthalat; Celluloseacetatphthalat und Ethylcellulose (am meisten bevorzugt mit einer Mischung im Verhältnis von 0 bis 6:1 von einem Copolymer im Verhältnis 1:2:0,1 von Ethylacrylat, Methylmethacrylat und Trimethylammoniumethylmethacrylatchlorid und einem Copolymer im Verhältnis 1:1 von Methylmethacrylat und Methacrylsäure, in einem Anteil von 5 bis 40 Gew.-%).

7. Vorrichtung nach irgendeinem der vorangegangenen Ansprüche, die zu einer oral verabreichbaren Form gefaltet ist ohne Beschränkungen, die ein elastisches Material aufweist.

8. Vorrichtung nach irgendeinem der vorangegangenen Ansprüche, die ein Arzneimittel trägt.

9. Vorrichtung nach Anspruch 8, die ein oder mehrere der folgenden Merkmale aufweist:
(a) das Arzneimittel ist Nicardipin;
(b) das Arzneimittel liegt in einer langzeitwirksamen Formulierung vor;
(c) das Arzneimittel liegt in Form einer Tablette oder dünnen Schicht vor.

10. Verfahren zum Herstellen einer Vorrichtung für eine zeitlich ausgedehnte Retention im Magen, wobei die Vorrichtung einen geformten Körper aus einem Material mit Gedächtniseffekt und einem im Magen erodierbaren Material, die miteinander verbunden oder vermischt sind, umfaßt, wobei der geformte Körper gefaltet sein und bleiben kann bei einem Bereich oder Bereichen aus Material mit Gedächtniseffekt ohne Beschränkungen, die ein elastisches Material aufweist, um eine Verabreichung zu vereinfachen, und wobei sich der geformte Körper im Magen in die gespeicherte ungefaltete Anordnung zurückfalten kann, in der der geformte Körper eine solche Größe, Gestalt und Dauerhaftigkeit aufweist, daß er im Magen zurückgehalten wird, ohne in diesem das Durchtreten von Nahrung zu blockieren, und wobei das erodierbare Material kontrolliert im Magen erodiert, um Vorrichtungsrückstande zu hinterlassen, die den Magen verlassen können,
welches umfaßt, einen Körper mit Gedächtniseffekt, der das Material mit Gedächtniseffekt umfaßt, zu bilden, den Körper mit Gedächtniseffekt neben einem oder mehreren Körpern aus dem im Magen erodierbaren Material anzuordnen und sämtliche Körper mit Klebemittel oder Dichtungsmittel und/oder durch mechanische Verbindung zu verbinden.

11. Verfahren zum Herstellen einer Vorrichtung für eine zeitlich ausgedehnte Retention im Magen, wobei die Vorrichtung einen geformten Körper aus einem Material mit Gedächtniseffekt und einem im Magen erodierbaren Material, die miteinander verbunden oder vermischt sind, umfaßt, wobei der geformte Körper gefaltet sein und bleiben kann bei einem Bereich oder Bereichen aus Material mit Gedächtniseffekt ohne Beschränkungen, die ein elastisches Material aufweist, um eine Verabreichung zu vereinfachen, und wobei sich der geformte Körper im Magen in die gespeicherte ungefaltete Anordnung zurückfalten kann, in der der geformte Körper eine solche Größe, Gestalt und Dauerhaftigkeit aufweist, daß er im Magen zurückgehalten wird, ohne in diesem das Durchtreten von Nahrung zu blockieren, und wobei das erodierbare Material kontrolliert im Magen erodiert, um Vorrichtungsrückstände zu hinterlassen, die den Magen verlassen können,
welches umfaßt, eine integrale Scheibe oder Platte mit radial aufeinanderfolgenden Bereichen aus dem Material mit Gedächtniseffekt und dem im Magen erodierbaren Material, gegebenenfalls mit dazwischen eingebrachtem Dichtungsmittel, zu formen und die Scheibe oder Platte zu schneiden, um eine Vorrichtung zu bilden, die eine Mehrzahl von sich durch diese Bereiche erstreckenden Armen hat.

12. Verfahren zum Herstellen einer Vorrichtung für eine zeitlich ausgedehnte Retention im Magen, wobei die Vorrichtung einen geformten Körper aus einem Material mit Gedächtniseffekt und einem im Magen erodierbaren Material, die miteinander verbunden oder vermischt sind, umfaßt, wobei der geformte Körper gefaltet sein und bleiben kann bei einem Bereich oder Bereichen aus Material mit Gedächtniseffekt ohne Beschränkungen, die ein elastisches Material aufweist, um eine Verabreichung zu vereinfachen, und wobei sich der geformte Körper im Magen in die gespeicherte ungefaltete Anordnung zurückfalten kann, in der der geformte Körper eine solche Größe, Gestalt und Dauerhaftigkeit aufweist, daß er im Magen zurückgehalten wird, ohne in diesem das Durchtreten von Nahrung zu blockieren, und wobei das erodierbare Material kontrolliert im Magen erodiert, um Vorrichtungsrückstände zu hinterlassen, die den Magen verlassen können,
welches umfaßt, das Material mit Gedächtniseffekt und das im Magen erodierbare Material zu mischen und die Mischung zu formen oder zu formen und zu schneiden, um einen Körper mit Gedächtniseffekt mit der erforderlichen Anordnung zu bilden.

13. Verfahren zum Herstellen einer Vorrichtung für eine zeitlich ausgedehnte Retention im Magen, wobei die Vorrichtung einen geformten Körper aus einem Material mit Gedächtniseffekt und einem im Magen erodierbaren Material, die miteinander verbunden oder vermischt sind, umfaßt, wobei der geformte Körper gefaltet sein und bleiben kann bei einem Bereich oder Bereichen aus Material mit Gedächtniseffekt ohne Beschränkungen, die ein elastisches Material aufweist, um eine Verabreichung zu vereinfachen, und wobei sich der geformte Körper im Magen in die gespeicherte ungefaltete Anordnung zurückfalten kann, in der der geformte Körper eine solche Größe, Gestalt und Dauerhaftigkeit aufweist, daß er im Magen zurückgehalten wird, ohne in diesem das Durchtreten von Nahrung zu blockieren, und wobei das erodierbare Material kontrolliert im Magen erodiert, um Vorrichtungsrückstände zu hinterlassen, die den Magen verlassen können,
welches umfaßt, zwischen Lagen des im Magen erodierbaren Materials eine diskontinuierliche Lage des Materials mit Gedächtniseffekt, die alleine den Magen verlassen kann, anzuordnen, die Anordnung zu formen, um eine Verbundscheibe oder -platte zu bilden und die Vorrichtung mit Gedächtniseffekt daraus auszuschneiden.

14. Verfahren nach Anspruch 13, in dem:
(a) die diskontinuierliche Lage in Form einzelner Streifen und/oder Fäden und/oder Inseln vorliegt, und/oder
(b) zwei oder mehrere solche Lagen aus Material mit Gedächtniseffekt einzeln zwischen dementsprechend drei oder mehreren solchen erodierbaren Lagen angeordnet werden.

15. Verfahren nach irgendeinem der Ansprüche 10 bis 14, in dem (a) die Substanz mit Gedächtniseffekt Polynorbornen oder Polyurethan und/oder (b) das im Magen erodierbare Material Polyethylenoxid oder Polyvinylalkohol ist.

16. Verfahren nach irgendeinem der Ansprüche 10 bis 15, das umfaßt, die Vorrichtung vollständig oder teilweise mit einer im Magensaft nur geringfügig löslichen oder unlöslichen Substanz zu überziehen.

17. Verfahren nach irgendeinem der Ansprüche 10 bis 16, das umfaßt, die Vorrichtung zu einer oral verabreichbaren Form zu falten ohne Beschränkungen, die ein elastisches Material aufweist.

18. Verfahren nach irgendeinem der Ansprüche 10 bis 17, das unfaßt, ein Arzneimittel der Vorrichtung hinzuzufügen oder ein Arzneimittel in das erodierbare Material einzubringen.

19. Verfahren nach Anspruch 18, auf das ein oder mehrere der folgenden Merkmale zutreffen:
(a) das Arzneimittel ist Nicardipin;
(b) das Arzneimittel liegt in einer langzeitwirksamen Formulierung vor;
(c) das Arzneimittel liegt in Form einer Tablette oder dünnen Schicht vor.

## Revendications

1. Un dispositif pour une rétention prolongée dans l'estomac comprenant un corps formé en un matériau à mémoire de forme et en un matériau capable de s'éroder gastriquement assemblés ou mélangés ensemble, le corps formé étant capable d'être et de rester plié en une région ou des régions du matériau à mémoire de forme sans contrainte d'un matériau élastique pour faciliter une administration et de revenir dans l'estomac à la configuration dépliée mémorisée dans laquelle il est d'une dimension, d'une forme et d'une durabilité telles qu'il est retenu dans l'estomac sans y bloquer le passage de nourriture, et le matériau capable de s'éroder, s'érodant de manière contrôlée dans l'estomac pour laisser des restes du dispositif qui peuvent être évacués de l'estomac.

2. Un dispositif conforme à la revendication 1, ayant au moins trois bras coplanaires s'étendant depuis un centre, le dispositif au, et/ou contigü au, centre où les bras se rejoignent étant construit en ledit matériau à mémoire de forme et les portions du côté extérieur des bras étant construites en ledit matériau capable de s'éroder gastriquement in vivo.

3. Un dispositif conforme à la revendication 1, comprenant au moins trois bras coplanaires s'étendant depuis le centre, chaque bras étant construit en ledit matériau à mémoire de forme disposé en forme de croisillon entre des couches dudit matériau capable de s'éroder gastriquement.

4. Un dispositif conforme à une quelconque revendication précédente, dans lequel (a) ladite substance à mémoire de forme est un polynorbornène ou un polyuréthanne, et/ou (b) ledit matériau capable de s'éroder gastriquement est un oxyde de polyéthylène ou un alcool polyvinylique.

5. Un dispositif conforme à une quelconque revendication précédente, qui est partiellement ou complètement revêtu d'une substance modérément soluble ou insoluble dans un suc gastrique.

6. Un dispositif conforme à la revendication 5, dans lequel ladite substance de revêtement est au moins un matériau choisi parmi un copolymère 1 : 2 : 0,1 d'acrylate d'éthyle, de méthacrylate de méthyle et de chlorure de méthacrylate triméthyl-ammonioéthyle, un copolymère 1 : 1 de méthacrylate de méthyle et d'acide méthacrylique ; un copolymère 1 : 1 d'acrylate d'éthyle et d'acide méthacrylique ; un copolymère 2 : 1 de méthacrylate de méthyle et d'acide méthacrylique ; un phthalate d'hydroxypropylméthyle cellulose ; un phthalate d'acétate de cellulose ; et l'éthylcellulose (préférentiellement un mélange de 0 à 6 : 1 d'un copolymère 1 : 2 : 0,1 d'acrylate d'éthyle, de méthacrylate de méthyle et de chlorure de méthacrylate de triméthylammonioéthyle et un copolymère 1 : 1 de méthacrylate de méthyle et d'acide méthacrylique, dans un rapport de 5 à 40 % en poids).

7. Un dispositif conforme à l'une quelconque revendication précédente, qui est plié selon une forme administrable oralement sans contrainte d'un matériau élastique.

8. Un dispositif conforme à l'une quelconque des revendications précédentes, portant un médicament.

9. Un dispositif conforme à la revendication 8, ayant une ou plusieurs des caractéristiques suivantes :
(a) le médicament est de la nicardipine ;
(b) le médicament est une formulation à libération prolongée;
(c) le médicament est en comprimés ou sous forme de couche mince.

10. Un procédé de fabrication d'un dispositif pour une rétention prolongée dans l'estomac, ledit dispositif comprenant un corps formé en un matériau à mémoire de forme et en un matériau capable de s'éroder gastriquement assemblés ou mélangés ensemble, le corps formé étant capable d'être et de rester plié en une région ou des régions du matériau à mémoire de forme sans contrainte d'un matériau élastique pour faciliter l'administration et de revenir dans l'estomac à la configuration dépliée mémorisée dans laquelle il est d'une dimension, d'une forme et d'une durabilité telles qu'il peut être retenu dans l'estomac sans y bloquer le passage de nourriture, et le matériau capable de s'éroder, s'érodant de manière contrôlée dans l'estomac pour laisser des restes du dispositif qui peuvent s'évacuer de l'estomac, qui comprend la mise en forme d'un corps à mémoire de forme comprenant ledit matériau à mémoire de forme, la juxtaposition du corps à mémoire de forme avec un ou plusieurs corps dudit matériau capable de s'éroder gastriquement et l'assemblage de tous les corps ensemble avec un matériau adhésif ou de liaison et/ou une liaison mécanique.

11. Un procédé de fabrication d'un dispositif pour une rétention prolongée dans l'estomac, ledit dispositif comprenant un corps formé en un matériau à mémoire de forme et en un matériau capable de s'éroder gastriquement combinés ou mélangés ensemble, le corps formé étant capable d'être et de rester plié en une région ou des régions du matériau à mémoire de forme sans contrainte d'un matériau élastique pour faciliter l'administration et de revenir dans l'estomac à la configuration dépliée mémorisée dans laquelle il est d'une dimension, d'une forme et d'une durabilité telles qu'il peut être retenu dans l'estomac sans y bloquer le passage de nourriture, et le matériau capable de s'éroder, s'érodant de manière contrôlée dans l'estomac pour laisser des restes du dispositif qui peuvent s'évacuer de l'estomac, qui comprend le moulage d'un disque ou d'une plaque d'une seule pièce ayant des régions successives radialement dudit matériau à mémoire de forme et dudit matériau capable de s'éroder gastriquement, optionnellement avec un matériau de liaison entre eux, et le découpage du disque ou de la plaque pour former un dispositif ayant une pluralité de bras s'étendant à travers lesdites régions.

12. Un procédé de fabrication d'un dispositif pour une rétention prolongée dans l'estomac, ledit dispositif comprenant un corps formé en un matériau à mémoire de forme et en un matériau capable de s'éroder gastriquement combinés ou mélangés ensemble, le corps formé étant capable d'être et de rester plié en une région ou des régions du matériau à mémoire de forme sans contrainte d'un matériau élastique pour faciliter une administration et de revenir dans l'estomac à la configuration dépliée mémorisée dans laquelle il est d'une dimension, d'une forme et d'une durabilité telles qu'il peut être retenu dans l'estomac sans y bloquer le passage de la nourriture, et le matériau capable de s'éroder s'érodant de manière contrôlée dans l'estomac pour laisser des restes du dispositif qui peuvent s'évacuer de l'estomac, qui comprend le mélange dudit matériau à mémoire de forme et dudit matériau capable de s'éroder gastriquement et le moulage, ou le moulage et le découpage, du mélange pour former un corps à mémoire de forme de la configuration requise.

13. Un procédé de fabrication d'un dispositif pour une rétention prolongée dans l'estomac, ledit dispositif comprenant un corps formé en un matériau à mémoire de forme et en un matériau capable de s'éroder gastriquement combinés ou mélangés ensemble, le corps formé étant capable d'être, et de rester, plié en une région ou des régions du matériau à mémoire de forme, sans contrainte d'un matériau élastique, pour faciliter une administration et de revenir dans l'estomac à la configuration dépliée mémorisée dans laquelle il est d'une dimension, d'une forme et d'une durabilité telles qu'il peut être retenu dans l'estomac sans y bloquer le passage de nourriture, et le matériau capable de s'éroder s'érodant de manière contrôlée dans l'estomac pour laisser des restes du dispositif qui peuvent s'évacuer de l'estomac, qui comprend la prise en sandwich entre des couches dudit matériau capable de s'éroder gastriquement d'une couche discontinue dudit matériau à mémoire de forme qui de lui-même peut sortir de l'estomac, le moulage de l'ensemble pour former un disque ou une plaque composite, et le découpage du dispositif à mémoire de forme en résultant.

14. Un procédé conforme à la revendication 13, dans lequel:
(a) ladite couche discontinue est sous forme de bandes séparées et/ou de fils et/ou d'îles et/ou
(b) deux ou plusieurs de telles couches d'un matériau à mémoire de forme sont prises en sandwich individuellement entre également trois ou plusieurs de telles couches capable de s'éroder.

15. Un procédé conforme à l'une quelconque des revendications 10 à 14, dans lequel (a) ladite substance à mémoire de forme est un polynorbornène ou un polyuréthanne et/ou (b) ledit matériau capable de s'éroder gastriquement est un oxyde de polyéthylène ou un alcool polyvinylique.

16. Un procédé conforme à l'une quelconque des revendications 10 à 15, qui inclut le revêtement complet ou partiel du dispositif par une substance modérément soluble ou insoluble dans un suc gastrique.

17. Un procédé conforme à l'une quelconque des revendications 10 à 16, qui inclut le pliage du dispositif sous une forme administrable oralement sans contrainte d'un matériau élastique.

18. Un procédé conforme à l'une quelconque des revendications 10 à 17, qui inclut l'application d'un médicament au dispositif ou l'incorporation d'un médicament dans le matériau capable de s'éroder.

19. Un procédé conforme à la revendication 18, dans lequel une ou plusieurs des caractéristiques suivantes s'appliquent :
(a) le médicament est de la nicardipine ;
(b) le médicament est dans une formulation à libération prolongée ;
(c) le médicament est en comprimés ou sous forme de couche mince.
